# EUROPEAN PATENT SPECIFICATION

(11) **EP 3 054 914 B1**
(45) Date of publication and mention of the grant of the patent: **08.12.2021**
(21) Application number: 13785941.9
(22) Date of filing: 11.10.2013
(51) Int. Cl.: A61J 15/00, G06F 9/00, A61M 5/142

(54) **ENTERAL FEEDING SYSTEM**
SYSTEM ZUR ENTERALEN ERNÄHRUNG
SYSTÈME D'ALIMENTATION ENTÉRALE

(43) Date of publication of application: 17.08.2016
(73) Proprietor: N.V. Nutricia, 2712 HM Zoetermeer (NL)
(72) Inventor: Zuijderduin, Bram, NL-3584 CT Utrecht (NL); Hogerwerf, Mark, NL-3584 CT Utrecht (NL)
(74) Representative: Nederlandsch Octrooibureau
(86) International application number: PCT/NL2013/050727
(87) International publication number: WO 2015/053616

(56) References cited:
- WO-A1-2010/054314
- US-A1- 2004 019 464
- US-B1- 8 021 322

## Description

### BACKGROUND OF THE INVENTION

### 1. Field of the Invention

The present invention relates to the enteral feeding of patients and to systems for performing such feeding. The invention further relates to an enteral pump and enteral feeding set and to a method of data management in the enteral feeding system. Such a device is disclosed in the US8021322B.

### 2. Description of the Related Art

For certain patients it is necessary to provide for their nutrition by enteral feeding. Enteral feeding generally refers to the delivery of a nutritionally complete feed, containing carbohydrates, proteins, fibre, fat, water, minerals and vitamins, directly into the stomach. In certain cases this may also be delivered to the duodenum or jejunum. Generally, delivery is via a naso-gastric tube, although it will be understood that other placements, including surgical e.g. percutaneous placements may be considered for long term care. Enteral feeding is to be distinguished from parenteral feeding which involves delivery of essential nutrients directly into the blood stream, bypassing completely the body's digestive apparatus.

Various enteral feeding solutions may be provided according to the specific nutritional requirements of the patient. These are usually pre-packaged in sterile reservoirs having an appropriate product label indicating the nutritional content. Feeding solutions may also be made up locally e.g. by a hospital pharmacy. Delivery takes place using a dedicated pump, usually a peristaltic type pump that acts on a section of the feeding tube to transfer the solution. Pumps may be portable or static and may be provided with various provisions for ensuring correct delivery and for monitoring the delivered volume. One such pump is known from WO201244860.

During feeding, it is desirable to closely monitor the nutritional intake status and fluid balance of the patient. In certain cases it is absolutely critical to be able to accurately monitor this throughout the enteral feeding therapy. At present, patient monitoring involves the healthcare professional manually collecting data from the product label and adding this information to the electronic medical record. Because of the nature of enteral feeding and the wide variation of the compositions from one solution to the next, each label contains a considerable quantity of data. The calculation of administered macro-nutrients (minerals, vitamins, spore elements, fat, proteins, etc.) as well as calories is then performed manually by integrating this information into a spreadsheet or other calculation method. This is time consuming and subject to mistakes. As a result, the fluid balance as well as the nutritional intake of the patient may be incorrectly monitored, which can have dangerous consequences for particular patients.

It would therefore be desirable to provide improved systems and devices which would facilitate the enteral feeding of patients and their correct monitoring and which may improve patient safety and user convenience.

### BRIEF SUMMARY OF THE INVENTION

According to the invention there is provided an enteral feeding system as further disclosed in claim 1, comprising: a reservoir including a quantity of a enteral feeding solution; a data carrier provided with nutritional data relating to the enteral feeding solution included in the reservoir; an enteral feeding set, connectable to the reservoir, for transferring the enteral feeding solution to a patient; a pump, operationally engageable with the enteral feeding set to cause transfer of the enteral feeding solution to the patient, the pump being provided with: a flow monitoring device adapted to monitor an amount of solution administered and generate flow data; a data reader, adapted to interrogate the data carrier to extract the nutritional data; and a patient monitoring module (PMM) adapted to interface with the pump and with the data reader to receive the flow data and the nutritional data, whereby real time delivery of individual nutrients can be calculated on the basis of the flow data and the nutritional data. As a result of the direct interrogation of the data carrier and the provision of this data to the PMM, the burden of manually entering data is reduced and accuracy is improved. Furthermore, by providing this data to the PMM, together with the flow data, the PMM can calculate the actual amount of a given nutrient received at any point in time without requiring a user to calculate the amount based on the fraction of a reservoir delivered.

The nutritional data includes the identity and quantity of each of the nutrients in the enteral feeding solution. For an enteral feeding solution, this nutritional data can be extensive. The nutritional data may include energy content, fat content, carbohydrate content, protein content, dietary fibre content, vitamin content, mineral content, osmolarity and osmolality. Most preferably, all of these are included and present on the label. Other nutrients may also be included in the enteral feeding solution and referenced on the label. In this context, nutrient is used generally to refer to all of the ingredients, whether or not they may actually be metabolised or otherwise taken up by the body. The nutritional data may also include the identity of the product, such as by name and batch number. Alternatively, the nutritional data may comprise merely an identification of the enteral feeding solution e.g. by name and batch number. In that case, additional nutritional data, such as the identity and quantity of each of the nutrients in the enteral feeding solution, may be provided or requested from another source, based on this identification. The other source from which the additional nutritional data may be gathered may be for example an online source such as a data library, webserver, data centre or the like.

The data carrier may be any appropriate element capable of providing the nutritional data to the data reader. In particular it may comprise a bar code, including 2D bar codes, datamatrix, QR-code and the like, an RFID tag or chip, and any other suitable optical, magnetic or electronic record carrier. The data carrier may be provided on an external package, label, card, tag or the like. Preferably, it is affixed to the reservoir e.g. in the form of an adhesive label or directly printed onto the packaging. The choice of data carrier may also depend on the form of the reservoir. In this context, it will be understood that the reservoir may include any conventional form of reservoir used for enteral feeding solutions, including both rigid and flexible containers such as bottles, boxes, flasks, pouches, bags, tubs and the like. The data carrier may alternatively be attached to the enteral feeding set, e.g. in the case that the reservoir and enteral feeding set are to be replaced together.

According to an important aspect of the invention, the system is arranged to provide at least 1000 bytes of nutritional information to the PMM. Typically 500 - 1500 bytes of data is required to adequately represent all of the nutritional data in an enteral feeding solution. This nutritional data must be made available to the PMM in order for it to perform its function.

According to one aspect of the invention, data compression may be used to reduce the amount of data transmitted from the data carrier to the PMM. In one preferred embodiment, the data carrier includes at least 30 bytes of compressed nutritional data and the data reader is adapted to transfer this data to the PMM, which extracts or otherwise decompresses the data to provide at least 1Kb of nutritional data. A suitable form of data compression may involve standard codes for each of the nutrients in the solution e.g., the text "Energy" consisting of 6 characters may be replaced by the characters AA, requiring only 2 bytes of data. Additionally, the units may be omitted from the transmission and only the values transmitted. The text: "Energy 103 kcal" can thus be converted to AA67 which is only 4 byte of data. The skilled person will understand that all of the nutritional data can be converted in this manner, reducing the space on a label required for this information and also reducing the data transmission. Other data compression methods may be used for providing the nutritional data on the data carrier and further optimization of the transmission may also be carried out. It will further be understood that in the case of data compression, the PMM will be provided with suitable decompression capability for reading and expanding the transmitted data to its full size.

In one particularly preferred embodiment, the pump and reservoir form part of an ambulatory system. In this case, the pump will be provided with its own internal energy source and may either have the PMM included within the ambulatory system or may communicate with a PMM at a remote location, e.g. by wireless means. In an alternative embodiment, the pump may be part of a stationary or bedside installation and may be physically connected for power and information exchange.

The pump may operate according to any customary method of operation suitable for enteral feeding purposes. Preferably it is arranged to have a disposable portion that is in contact with the enteral feeding solution. Most preferably the pump is a peristaltic, roller or diaphragm pump. The flow monitoring device may be any suitable means for determining the flow rate through the pump. This may include direct flow monitoring, which measures the actual flow of enteral feeding solution within the enteral feeding set, and indirect flow monitoring, which may measure the speed of operation of the pump. In one embodiment, the pump is a roller pump having a rotor and the flow monitoring device measures the number or rotations or part rotations of the rotor.

In general, the enteral feeding set will depend upon the type of pump being used. Preferably, the enteral feeding set comprises a reservoir connector for connection to the reservoir, a flexible tubing of sufficient length to bridge the distance between the reservoir and the patient, a captive pump insert which interacts with the pump to cause pumping of the enteral feeding solution, an injection gate to allow additional drug or fluid administration and a distal end connector to connect to an invasive enteral feeding tube or any suitable patient delivery portion such as a nasogastric tube, nasoduodenal tube, nasojejunal tube, gastrostomy feeding tube, gastrojejunostomy feeding tube or jejunostomy feeding tube.

The PMM may be implemented either as software or hardware or a combination thereof, to the extent that it can perform the required function of interfacing with the pump and with the data reader to receive the flow data and the nutritional data in order to calculate the real time delivery of individual nutrients on the basis of the flow data and the nutritional data. It can also be located at any position in the system or may be distributed. As indicated above, the PMM may in one embodiment be remotely located from the pump and communication may take place through an appropriate communication channel, such as wirelessly or over the internet. Alternatively, the PMM may be integrated within the pump itself, e.g. as a module forming part of a controller of the pump.

In addition to the PMM, the enteral feeding system may further comprise a patient data monitoring system (PDMS) arranged to store patient data relating to the patient and to a plurality of further patients. The PMM is preferably arranged to interface with the PDMS to update the patient data and the skilled person will understand that it may have all of the necessary drivers required to interface therewith. Updating may take place on the basis of the real time delivery of the various individual nutrients as calculated by the PMM. In one particular embodiment, the PDMS is arranged to calculate the real time delivery of individual nutrients on the basis of the flow data and the nutritional data and further on the basis of library data received from an electronic library external to the system. The library data may be provided to the PDMS or may be provided directly to the PMM. In general, the latter may be preferred and the library may be dedicated to a particular PMM including information related to the specific pump and enteral feeding solutions. According to one embodiment, the library data comprises data relating to the identity and quantity of each of the nutrients in the enteral feeding solution and the library data is received in response to a request for data based on the nutritional data. It will also be understood that the PDMS may itself be connected to a further library for the provision of other data such as clinical data from other locations within an institution or from a healthcare provider or doctor.

According to the invention, the system may be provided with a display for displaying the calculated real time delivery of the individual nutrients. The display may form part of the PDMS although other display locations may also be provided. Preferably the display and its associated drivers allows for real time monitoring of all or any of the individual nutrients, individually or together. Preferably the display allows a graphical representation of both delivery rate and cumulative delivery over a time period. Because significant variations can occur between the relative quantities of different nutrients, the display may provide for logarithmic representation of the respective nutrients or use different axes for different nutrients.

According to a further aspect of the invention, the pump comprises an occlusion detector arranged to allow operation of the pump up to an occlusion pressure of at least 50 KPa, preferably at least 60 KPa and most preferably at least 80 KPa. In general, enteral feeding solutions are relatively viscous and the pump must exert considerable pressure in order to pump the solution to the body, even when no occlusion occurs. In the event that the enteral feeding set is bent or blocked, excess pressure may occur and in order to avoid false alarms, the occlusion detector may be set to a relatively high value before an alarm is given. Values as high as 200 Kpa (2 bar) may even be permitted. The enteral feeding solution may have a viscosity of at least of 0.1 Pa.s but preferably will have a viscosity of between 1 Pa.s and 250 Pa.s, measured at shear rate of 100 s-1 at 20 oC, for example using the AR 2000 EX by TA-instruments.

The invention also relates to a method of data management in an enteral feeding system, comprising: extracting nutritional data from a data carrier relating to the nutritional composition of an enteral feeding solution; delivering the enteral solution to a patient; monitoring delivery of the enteral feeding solution to generate flow data based on an amount of solution delivered; and calculating a real time delivery of individual nutrients on the basis of the flow data and the nutritional data.

The method may also include displaying the real time delivery of a plurality of individual nutrients on a display of a patient data monitoring system (PDMS). In this manner, a care giver can at any moment determine the state of administration of the relevant nutrient.

Additionally, the method may comprise decompressing the nutritional data received from the data carrier prior to calculating the real time delivery, whereby the real time delivery is calculated on the basis of the decompressed data.

The invention still further relates to an enteral feeding pump for use in an enteral feeding system, comprising: a pump, operationally engageable with an enteral feeding set to cause transfer of an enteral feeding solution from a reservoir to a patient, the pump being provided with a flow monitoring device adapted to monitor an amount of solution administered and generate flow data; a data reader, adapted to interrogate a data carrier to extract nutritional data relating to a nutritional content of the enteral feeding solution; a patient monitoring module (PMM) adapted to interface with the pump and with the data reader to receive the flow data and the nutritional data and calculate a real time delivery of individual nutrients on the basis of the flow data and the nutritional data.

### BRIEF DESCRIPTION OF THE DRAWINGS

The features and advantages of the invention will be appreciated upon reference to the following drawings of a number of exemplary embodiments, in which:
Figure 1 shows a schematic view of an enteral feeding system according to the present invention;
Figure 2 shows the label of Figure 1;
Figure 3 shows a screen shot of the PDMS in a first display mode; and
Figure 4 shows a screen shot of the PDMS in a second display mode.

### DESCRIPTION OF ILLUSTRATIVE EMBODIMENTS

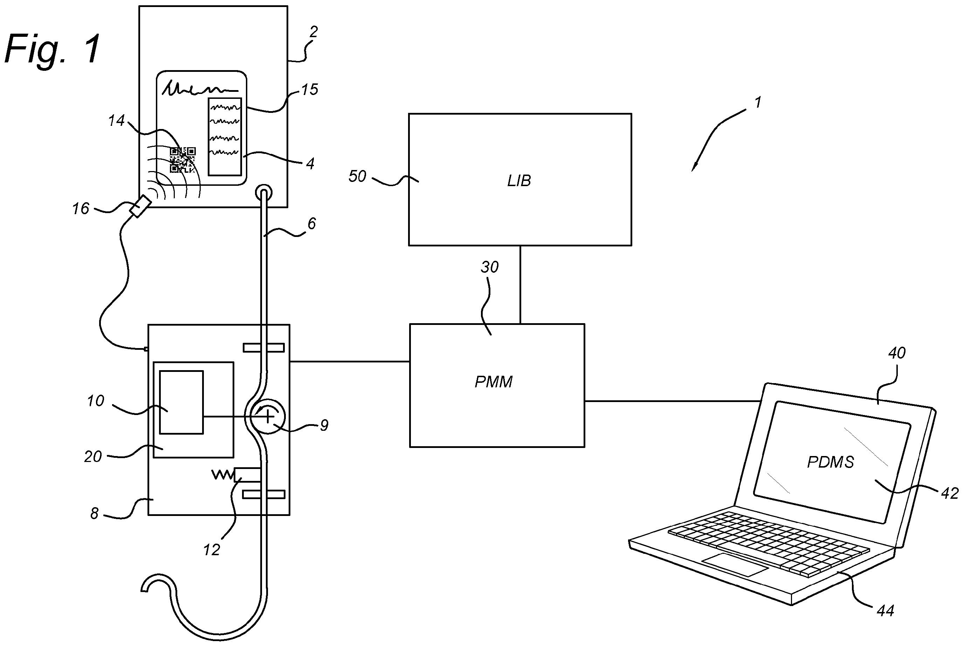

Figure 1 shows a schematic view of an enteral feeding system 1 according to the present invention. The enteral feeding system 1 comprises a reservoir 2 including a quantity of an enteral feeding solution 4. The reservoir 2 is a generally conventional flexible pouch and is releasably connected to an enteral feeding set 6 for transferring the enteral feeding solution 4 to a patient (not shown). For administration of the enteral feeding solution 4, a pump 8 is provided. The pump 8 is a Nutricia FlocareTM enteral feeding roller pump with a disposable pump insert on which a rotor 9 of the pump 8 acts. The skilled person will nevertheless understand that other forms of pump, enteral feeding set and reservoir could be used within the scope of the invention. The pump 8 is operationally engaged with the enteral feeding set 6 to cause transfer of the enteral feeding solution 4 to the patient. The pump 8 includes a flow monitoring device 10 adapted to monitor the rotation of the rotor 9 to determine the amount of enteral solution 4 administered and generate flow data based on a real time evaluation of the fluid administered. The pump 8 is also provided with appropriate sensors as may otherwise be conventional, including an occlusion sensor 12. The occlusion sensor 12 is set to a pressure value which can give warning to a user in the event of an occlusion downstream of the pump. It will be understood that enteral feeding solutions are of relatively high viscosity and a value of around 83 KPa +-21 kPa is generally conventional for such sensors in order to avoid false alarms. Additional upstream occlusion sensing may also be provided.

According to the invention, the reservoir 2 is provided with a data carrier 14 provided with nutritional data relating to the enteral feeding solution 4. This is in the form of a large format 2D barcode, containing around 500-1500B of data. The data carrier 14 is provided on a label 15 carrying similar data in readable form. The skilled person will understand that other forms of data carriers could be used subject to them being able to store sufficient data representative of the enteral feeding solution 4 in the reservoir 2. Also provided is a data reader 16, adapted to interrogate the data carrier 14 to extract the nutritional data. The data reader 16 is a hand-held barcode scanner operationally connected to the pump 8 for transmission of data thereto. It is alternatively envisaged that the data reader may be in the form of an integrated scanner provided on the pump body.

Figure 1 also shows a pump controller 20, a patient monitoring module (PMM) 30, a patient data management system (PDMS) 40 and a data library 50, adapted to interface with each other as will be explained in further detail below. The pump controller 20 is integrated as part of the pump 8 and interacts with the flow monitoring device 10, occlusion sensor 12 and other sensors of the pump 8. The PMM 30, the PDMS 40 and the data library 50 are external modules, remote from the pump 8. Nevertheless, the skilled person will recognise that some or all of the functions of these modules may also be distributed elsewhere, such as within the pump 8 itself. The communication between the pump controller 20 and the external modules in the illustrated embodiment is a wired connection. It will however be understood that the communication may also take place e.g. over the internet or by dedicated secure communication methods

In the illustrated embodiment, the PDMS 40 is provided with a display 42 and a user interface 44. It will be understood that the PMM 30 may also be provided with a display and user interface if so required e.g. for bedside use.

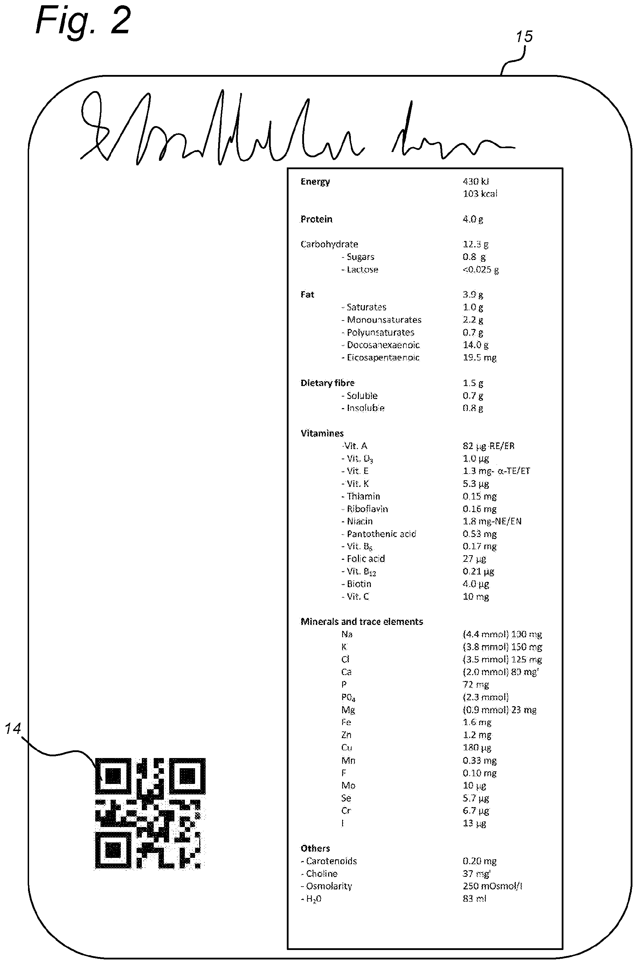

Figure 2 shows the label 15 of Figure 1 including the nutritional data in readable form and the data carrier 14, which incorporates the same data in compressed form. This nutritional data includes the identity and quantity of each of the nutrients in the enteral feeding solution 4, normalised per 100 ml of the solution. Although not shown, it may also include the name, expiry date, logistic information and batch number of the enteral feeding solution 4.

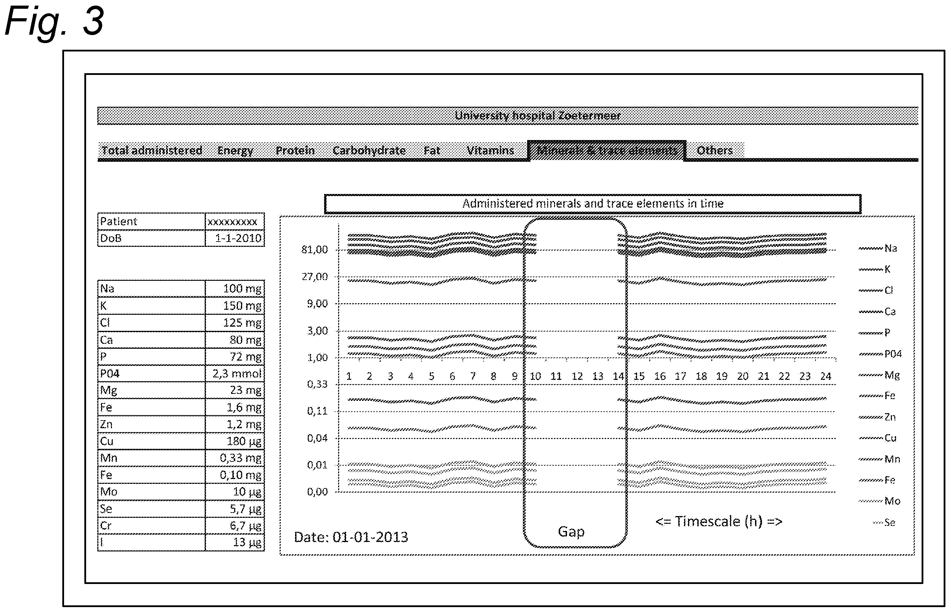

Figure 3 shows a screen-shot of the PDMS display 42 in a view of the minerals and trace elements display.

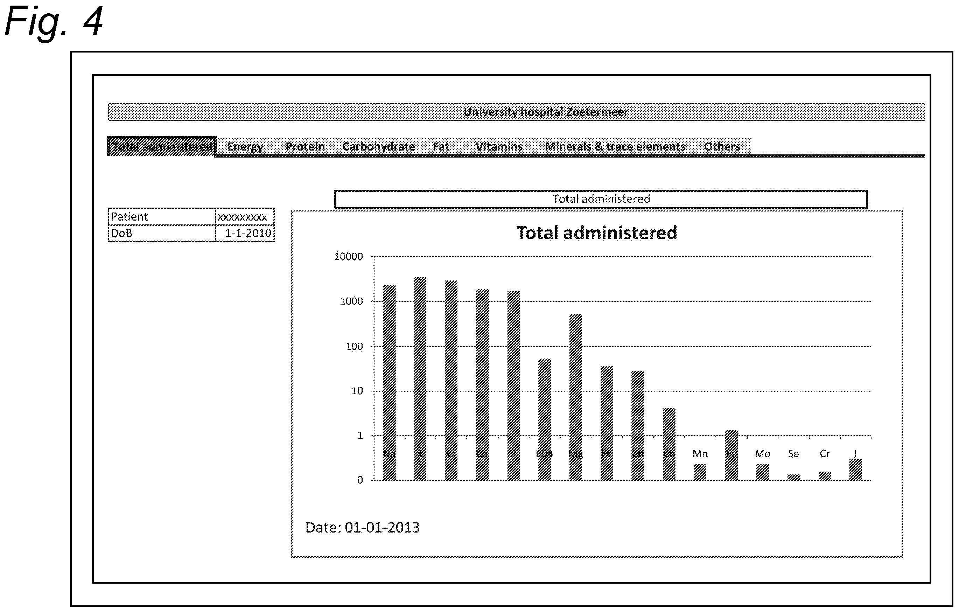

Figure 4 shows a screen-shot of the PDMS display 42 in a view of the total administered screen for minerals and trace elements.

Operation of the enteral feeding system 1 will be described with reference to Figures 1 to 4. In use, a user or care-giver wishing to provide enteral feeding, connects enteral feeding set 6 to a new reservoir 2 of enteral feeding solution 4. Once connected, and with the enteral feeding set 6 inserted in the pump 8, the user scans the data carrier 14 with the data reader 16. The nutritional data incorporated on the data carrier 14 is extracted from the data carrier 14 and passed to the PMM 30. PMM 30 decompresses the compressed data into the form as provided on the label 15.

The pump 8 is set into operation and under the control of the pump controller 20, commences delivery of the enteral feeding solution 4 to the patient at a nominal rate of 100 ml per hour. Flow monitoring device 10 records the flow rate based on the number of rotations of rotor 9 and generates real-time flow data which is also provided to the PMM 30. On the basis of the flow data and the nutritional data, PMM 30 calculates a real time delivery of nutrients to the patient. This delivery data may comprise a momentary rate of delivery based on the concentration of an individual nutrient in the solution 4 multiplied by the momentary flow rate. It can also include the cumulative delivery based on an integration of the flow rate over time and the relevant concentration. The PMM transmits the respective delivery data to the PDMS 40 where it is displayed to a care-giver on display 42. In addition to the delivery data received from the PMM 30, the care giver may also extract data from the data library 50 to further expand the patient record. Such data may include additional drug data or nutritional data extracted from the data library 50 on the basis of the name and batch number collected from the data carrier 14. The care-giver may also input data, such as patient data and additional drug or nutritional data, directly via the user interface 44.

In Figure 3, the rate of administration of the minerals and trace elements to patient XXXX over time is shown. In the illustrated example, all minerals are shown on a single graph over the previous 24 hour period with a scale of mg/hour given on the y-axis in a logarithmic scale. It can be seen that the momentary delivery rate at hour 24 for Mg is around 23 mg/hour. The care giver can also observe that at around hour 10 the delivery has been stopped and has restarted at hour 14. It will be understood that for the sake of clarity, separate displays for each nutrient may be requested, each on an appropriate scale. The user can also choose to display values for energy, proteins, carbohydrates, fats or vitamins. Alternatively, a number of nutrients may be selected from a list for display together.

In Figure 4, the user has chosen to display the momentary total cumulative delivery for minerals and trace elements. This is the total in mg of the respective nutrient delivered over the last 24 hour period. A care giver can directly determine how much of a particular nutrient has been taken and may take this into account on assessing the condition of a patient and whether any additional nutrition may be required. Thus, the invention has been described by reference to certain embodiments discussed above. It will be recognized that these embodiments are susceptible to various modifications and alternative forms well known to those of skill in the art. In particular, the particular data transferred and displayed will depend upon the treatment regime and may vary accordingly.

## Claims

1. An enteral feeding system comprising:
a reservoir (2) including a quantity of an enteral feeding solution comprising a plurality of individual nutrients;
a data carrier (14) provided with nutritional data relating to the identity and quantity of each of the individual nutrients of the enteral feeding solution included in the reservoir;
an enteral feeding set, connectable to the reservoir, for transferring the enteral feeding solution to a patient;
a pump (8), operationally engageable with the enteral feeding set to cause transfer of the enteral feeding solution to the patient, the pump being provided with a flow monitoring device adapted to monitor an amount of solution administered and generate flow data;
a data reader (16), adapted to interrogate the data carrier to extract the nutritional data; and
a patient monitoring module (PMM) (30) adapted to interface with the pump and with the data reader to receive the flow data and the nutritional data, whereby real time delivery of the individual nutrients can be calculated on the basis of the flow data and the nutritional data.

2. The system according to claim 1, wherein the nutritional data includes the identity and quantity of each of the nutrients in the enteral feeding solution.

3. The system according to claim 1 or claim 2, wherein the data carrier is affixed to the reservoir.

4. The system according to any preceding claim, wherein the data carrier includes at least 30 byte of nutritional data and the PMM is adapted to decompress or otherwise extract the data to provide at least 1Kb of nutritional data.

5. The system according to any preceding claim, wherein the data carrier comprises a 2-D barcode.

6. The system according to any preceding claim, wherein the PMM is integrated in the pump.

7. The system according to any preceding claim, wherein the PMM is remotely located from the pump and communication takes place wirelessly.

8. The system according to any preceding claim, further comprising a patient data monitoring system (PDMS) arranged to store patient data relating to the patient and to a plurality of further patients and wherein the PMM interfaces with the PDMS to update the patient data, wherein the PDMS is preferably remotely located from the pump and communication takes place wirelessly.

9. The system according to any preceding claim, wherein the PMM is arranged to calculate the real time delivery of individual nutrients on the basis of the flow data and the nutritional data and further on the basis of library data received from an electronic library external to the system.

10. The system according to claim 9, wherein the library data comprises data relating to the identity and quantity of each of the nutrients in the enteral feeding solution and the library data is received in response to a request for data based on the nutritional data.

11. The system according to any preceding claim, wherein the pump comprises an occlusion detector arranged to allow operation of the pump up to an occlusion pressure of at least 50 KPa, preferably at least 60 KPa and most preferably at least 80 KPa.

12. The system according to any preceding claim, wherein the enteral feeding solution has a viscosity of at least 0.1 Pa.S and preferably between 1 Pa.S and 250 Pa.S. measured at shear rate of 100 s-1 at 20 °C.

13. A method of data management in an enteral feeding system according to claim 1, comprising:
extracting nutritional data from a data carrier relating to the identity and quantity of each of the individual nutrients of a plurality of individual nutrients in an enteral feeding solution;
delivering the enteral feeding solution through an enteral feeding set;
monitoring delivery of the enteral feeding solution to generate flow data based on an amount of solution delivered through the feeding set;
calculating a real time delivery of the individual nutrients on the basis of the flow data and the nutritional data.

14. The method of claim 13, further comprising displaying the real time delivery of the plurality of individual nutrients on a display of a patient data monitoring system (PDMS).

15. The method of claim 13 or claim 14, further comprising decompressing the nutritional data received from the data carrier.

## Patentansprüche

1. System zur enteralen Ernährung, das aufweist:
ein Reservoir (2), das eine Quantität einer Lösung zur enteralen Ernährung umfasst, die eine Vielzahl individueller Nährstoffe aufweist;
einen Datenträger (14), der mit Nährstoffdaten versehen ist, die sich auf die Identität und die Quantität eines jeden der individuellen Nährstoffe der Lösung zur enteralen Ernährung beziehen, die in dem Reservoir umfasst ist;
ein Set zur enteralen Ernährung, das mit dem Reservoir verbindbar ist, zum Weiterleiten der Lösung zur enteralen Ernährung an einen Patienten;
eine Pumpe (8), die zum Veranlassen eines Weiterleitens der Lösung zur enteralen Ernährung bedienbar mit dem Set zur enteralen Ernährung in Eingriff bringbar ist, wobei die Pumpe mit einer Strömungsüberwachungsvorrichtung versehen ist, die dazu angepasst ist, eine Menge einer verabreichten Lösung zu überwachen und Strömungsdaten zu erzeugen;
einen Datenleser (16), der dazu angepasst ist, den Datenträger zum Extrahieren der Nährstoffdaten abzufragen; und
ein Patientenüberwachungsmodul (PMM) (30), das dazu angepasst ist, sich zum Empfangen der Strömungsdaten und der Nährstoffdaten mit der Pumpe und dem Datenleser zu koppeln, wodurch eine Echtzeitzuführung der individuellen Nährstoffe auf der Grundlage der Strömungsdaten und der Nährstoffdaten berechnet werden kann.

2. System gemäß Anspruch 1, wobei die Nährstoffdaten die Identität und die Quantität eines jeden der Nährstoffe in der Lösung zur enteralen Ernährung umfasst.

3. System gemäß Anspruch 1 oder 2, wobei der Datenträger an dem Reservoir befestigt ist.

4. System gemäß einem der vorangehenden Ansprüche, wobei der Datenträger mindestens 30 byte Nährstoffdaten umfasst und das PPM dazu angepasst ist, die Daten zu dekomprimieren oder anderweitig zu extrahieren, sodass es mindestens 1 Kb Nährstoffdaten zur Verfügung stellt.

5. System gemäß einem der vorangehenden Ansprüche, wobei der Datenträger einen 2-D-Barcode aufweist.

6. System gemäß einem der vorangehenden Ansprüche, wobei das PPM in der Pumpe integriert ist.

7. System gemäß einem der vorangehenden Ansprüche, wobei das PPM von der Pumpe entfernt angeordnet ist und eine Kommunikation kabellos stattfindet.

8. System gemäß einem der vorangehenden Ansprüche, das ferner ein Patientendatenüberwachungssystem (PDMS) aufweist, das dazu eingerichtet ist, Patientendaten zu speichern, die sich auf den Patienten und auf eine Vielzahl weiterer Patienten beziehen, und wobei sich das PPM zum Aktualisieren der Patientendaten mit dem PDMS koppelt, wobei das PDMS vorzugsweise von der Pumpe entfernt angeordnet ist und eine Kommunikation kabellos stattfindet.

9. System gemäß einem der vorangehenden Ansprüche, wobei das PMM dazu eingerichtet ist, die Echtzeitzuführung von individuellen Nährstoffen auf der Grundlage der Strömungsdaten und der Nährstoffdaten und ferner auf der Grundlage von Datenbankdaten zu berechnen, die von einer elektronischen Datenbank empfangen werden, die außerhalb des Systems ist.

10. System gemäß Anspruch 9, wobei die Datenbankdaten Daten aufweisen, die sich auf die Identität und die Quantität eines jeden der Nährstoffe in der Lösung zur enteralen Ernährung beziehen, und die Datenbankdaten als Antwort auf eine Datenanfrage auf Grundlage der Nährstoffdaten empfangen werden.

11. System gemäß einem der vorangehenden Ansprüche, wobei die Pumpe eine Verschlusserfassung aufweist, die dazu eingerichtet ist, einen Betrieb der Pumpe bis zu einem Verschlussdruck von mindestens 50 KPa, bevorzugt mindestens 60 KPa und am meisten bevorzugt mindestens 80 KPa zu erlauben.

12. System gemäß einem der vorangehenden Ansprüche, wobei die Lösung zur enteralen Ernährung eine bei einer Scherrate von 100 s-1 bei 20°C gemessene Viskosität von mindestens 0,1 Pa.S und vorzugsweise zwischen 1 Pa.S und 250 Pa.S hat.

13. Verfahren eines Datenmanagements bei einem System zur enteralen Ernährung gemäß Anspruch 1, das die folgenden Schritte aufweist:
Extrahieren von Nährstoffdaten von einem Datenträger, die sich auf die Identität und die die Quantität eines jeden der individuellen Nährstoffe einer Vielzahl individueller Nährstoffe in einer Lösung zur enteralen Ernährung beziehen;
Zuführen der Lösung zur enteralen Ernährung über ein Set zur enteralen Ernährung;
Überwachen einer Zuführung der Lösung zur enteralen Ernährung zum Erzeugen von Strömungsdaten auf Grundlage einer über das Set zur Ernährung zugeführten Lösungsmenge;
Berechnen einer Echtzeitzuführung der individuellen Nährstoffe auf der Grundlage der Strömungsdaten und der Nährstoffdaten.

14. Verfahren gemäß Anspruch 13, das ferner einen Schritt zum Anzeigen der Echtzeitzuführung der Vielzahl individueller Nährstoffe an einer Anzeige eines Patientendatenüberwachungssystems (PDMS) aufweist.

15. Verfahren gemäß Anspruch 13 oder 14, das ferner einen Schritt zum Dekomprimieren der von dem Datenträger empfangenen Nährstoffdaten aufweist.

## Revendications

1. Système d'alimentation entérale comprenant :
un réservoir (2) incluant une quantité d'une solution d'alimentation entérale comprenant une pluralité de nutriments individuels ;
un support de données (14) muni de données nutritionnelles concernant l'identité et la quantité de chacun des nutriments individuels de la solution d'alimentation entérale incluse dans le réservoir ;
un ensemble d'alimentation entérale, pouvant être connecté au réservoir, pour transférer la solution d'alimentation entérale à un patient ;
une pompe (8), pouvant être mise en prise de manière opérationnelle avec l'ensemble d'alimentation entérale pour provoquer un transfert de la solution d'alimentation entérale au patient, la pompe étant pourvue d'un dispositif de surveillance d'écoulement adapté pour surveiller une quantité de solution administrée et générer des données d'écoulement ;
un lecteur de données (16), adapté pour interroger le support de données pour extraire les données nutritionnelles ; et
un module de surveillance de patient (PMM) (30) conçu pour servir d'interface avec la pompe et avec le lecteur de données pour recevoir les données d'écoulement et les données nutritionnelles, moyennant quoi une distribution en temps réel des nutriments individuels peut être calculée sur la base des données d'écoulement et des données nutritionnelles.

2. Système selon la revendication 1, dans lequel les données nutritionnelles incluent l'identité et la quantité de chacun des nutriments dans la solution d'alimentation entérale.

3. Système selon la revendication 1 ou la revendication 2, dans lequel le support de données est fixé au réservoir.

4. Système selon l'une quelconque des revendications précédentes, dans lequel le support de données inclut au moins 30 octets de données nutritionnelles et le PMM est adapté pour décompresser ou extraire autrement les données pour fournir au moins 1 Kb de données nutritionnelles.

5. Système selon l'une quelconque des revendications précédentes, dans lequel le support de données comprend un code à barres 2D.

6. Système selon l'une quelconque des revendications précédentes, dans lequel le PMM est intégré dans la pompe.

7. Système selon l'une quelconque des revendications précédentes, dans lequel le PMM est situé à distance de la pompe et la communication a lieu sans fil.

8. Système selon l'une quelconque des revendications précédentes, comprenant en outre un système de surveillance de données de patient (PDMS) agencé pour stocker des données de patient relatives au patient et à une pluralité de patients supplémentaires et dans lequel le PMM s'interface avec le PDMS pour mettre à jour les données de patient, dans lequel le PDMS est de préférence situé à distance de la pompe et la communication a lieu sans fil.

9. Système selon l'une quelconque des revendications précédentes, dans lequel le PMM est agencé pour calculer la distribution en temps réel de nutriments individuels sur la base des données d'écoulement et des données nutritionnelles et en outre sur la base de données de bibliothèque reçues d'une bibliothèque électronique externe au système.

10. Système selon la revendication 9, dans lequel les données de bibliothèque comprennent des données relatives à l'identité et à la quantité de chacun des nutriments dans la solution d'alimentation entérale et les données de bibliothèque sont reçues en réponse à une demande de données basée sur les données nutritionnelles.

11. Système selon l'une quelconque des revendications précédentes, dans lequel la pompe comprend un détecteur d'occlusion agencé pour permettre le fonctionnement de la pompe jusqu'à une pression d'occlusion d'au moins 50 KPa, de préférence d'au moins 60 KPa et de la manière la plus préférée d'au moins 80 KPa.

12. Système selon l'une quelconque des revendications précédentes, dans lequel la solution d'alimentation entérale a une viscosité d'au moins 0,1 Pa.S et de préférence comprise entre 1 Pa.S et 250 Pa.S, mesurée à un taux de cisaillement de 100 s-1 à 20°C.

13. Procédé de gestion de données dans un système d'alimentation entérale selon la revendication 1, comprenant les étapes suivantes :
extraire des données nutritionnelles à partir d'un support de données, concernant l'identité et la quantité de chacun des nutriments individuels d'une pluralité de nutriments individuels dans une solution d'alimentation entérale ;
distribuer la solution d'alimentation entérale à travers un ensemble d'alimentation entérale ;
surveiller la distribution de la solution d'alimentation entérale pour générer des données d'écoulement sur la base d'une quantité de solution distribuée à travers l'ensemble d'alimentation ;
calculer une distribution en temps réel des nutriments individuels sur la base des données d'écoulement et des données nutritionnelles.

14. Procédé selon la revendication 13, comprenant en outre l'affichage de la distribution en temps réel de la pluralité de nutriments individuels sur un affichage d'un système de surveillance de données de patient (PDMS).

15. Procédé selon la revendication 13 ou la revendication 14, comprenant en outre une décompression des données nutritionnelles reçues à partir du support de données.
